# EUROPEAN PATENT SPECIFICATION

(11) **EP 1 811 616 B1**
(45) Date of publication and mention of the grant of the patent: **04.01.2017**
(21) Application number: 06021833.6
(22) Date of filing: 18.10.2006
(51) Int. Cl.: H01S 3/067, G02B 6/02, G02B 6/036, G02B 6/14, G02B 6/26

(54) **Rare-earth-doped, large-mode-area, multimode, hybrid optical fibers and devices using the same**
Mit seltenen Erden dotierten vielfachmodalen hybrid optische Fazer und Vorrichtungen
Fibres optiques du type multi-mode dopées aux terres rares et dispositifs

(30) Priority: 16.12.2005 US 750967 P
(43) Date of publication of application: 25.07.2007
(73) Proprietor: OFS Fitel, LLC, Norcross, GA 30071 (US)
(72) Inventor: Windeler, Robert S., Annadale, New Jersey 08801 (US); Yablon, Andrew D., Livingston, New Jersey 07039 (US)
(74) Representative: Zimmermann, Tankred Klaus

(56) References cited:
- WO-A1-2004/112207
- WO-A1-2005/041367
- WO-A2-02/39552
- US-A1- 2002 018 630
- US-A1- 2002 168 139
- US-A1- 2005 122 574
- US-A1- 2005 147 369
- US-B1- 6 324 326
- WALTER A. WEBER: "Der Whitehouse-Strehl-Faktor: ein wichtiger (aber wenig beachteter) Laserstrahlparameter", PHOTONIK, vol. 2, 1998, pages 6-9,

## Description

### BACKGROUND OF THE INVENTION

### Field of the Invention

This invention relates to optical fibers and, more particularly, to rare-earth-doped, large-mode-area, multimode optical fibers for high power optical amplifier or laser applications and improved coupling efficiency.

### Discussion of the Related Art

Because of their high performance and cost effectiveness, rare-earth-doped fiber amplifiers (REDFAs), especially erbium-doped fiber amplifiers (EDFAs), are widely used in silica fiber-optic communication systems such as, for example, long-haul transport and CATV applications. Innovative design and optimization of rare-earth-doped fibers (REDFs), especially erbium-doped fibers (EDFs), have both played a critical role in these applications. In particular, designs that confine the optical mode field and control the erbium distribution enable efficient, low-noise amplification of light at low and medium optical power levels. On the other hand, for high power applications large-mode-area (LMA) fiber with low numeric aperture (NA) lowers the signal intensity, thereby reducing deleterious nonlinear effects, and also increases the pump absorption efficiency. High power REDFAs and rare-earth doped fiber lasers (REDFLs), especially those utilizing ytterbium-doped fibers (YDFs), also have many applications outside the traditional telecommunications industry. For example, high power, LMA, YDFs are used in welding and cutting, laser ranging and target designation, medical applications and pollution detection, and free space communications (e.g., between satellites).

The optical characteristics of a LMA fiber sensitively depend upon the details of its transverse refractive index profile. Conventional wisdom dictates that desirable LMA fibers have a fundamental mode with M² very near to 1.0, meaning that the optical field of the fundamental transverse mode is very nearly Gaussian in shape because the refractive index profile is essentially uniform over the axial cross-section of the core. M² measures the similarity between the mode field and a true Gaussian function. More specifically, M² = 1.0 for a mode having a Gaussian shape, and M² > 1.0 for all other mode field shapes. An M² very near to 1.0 facilitates low loss optical coupling, and furthermore the beam emerging from the fiber may be efficiently collimated or tightly focused to a diffraction limited spot. However, fabricating an LMA fiber with an ideal fundamental mode (M² = 1.0) and a uniform core refractive index profile can be difficult due to the tendency of the profile to exhibit a dip in refractive index near the longitudinal axis (also known as a *center dip* or *burnoff*). Moreover, LMA fibers with a fundamental transverse mode M² very near to 1.0 exhibit smaller effective areas and hence lower thresholds for undesirable optical nonlinearities than the fundamental transverse modes of fibers with similar core diameters but pronounced center dips. Finally, when a LMA EDF's core transverse refractive index profile is essentially uniform and the fundamental mode's M² is very near to 1.0, there is relatively little overlap between the fundamental mode and the outer region of the doped core. As a result, the fundamental mode may experience low amplification efficiency while high-order modes may experience undesirable amplification.

Thus, a need remains in the art for a LMA REDF with improved optical coupling efficiency.

There is also a need for such a LMA REDF that is suitable for high power optical fiber amplifier and laser applications.

US 6,324,326 B1 and US 2002/0018630 A! disclose related rare earth doped fibre amplifiers or fibre lasers where the fundamental mode does not have a Gaussian shape (M²> 1.0).

US2005/0147369 A1 discloses a mode converting fibre patch cord without rare earth doping.

### BRIEF SUMMARY OF THE INVENTION

In accordance with one aspect of our invention, a LMA REDF is configured to support multiple transverse modes of signal radiation within its core region. Our fiber is a hybrid design that includes at least two axial segments having significantly different characteristics. In a first axial segment the transverse refractive index profile is not radially uniform, being characterized by a radial dip in refractive index. The first segment supports more than one transverse mode. In a second axial segment the transverse refractive index profile is more uniform than that of the first segment. The two segments are adiabatically coupled to one another. Illustratively, the second segment is a terminal portion of the fiber which facilitates coupling to other components.

In one embodiment of our invention, in the first segment M₁² > 1.0, and in the second segment M₂² << M₁². In a preferred embodiment, M₁²>> 1.0 and M₂² ∼ 1.0.

### BRIEF DESCRIPTION OF THE SEVERAL VIEWS OF THE DRAWING

Our invention, together with its various features and advantages, can be readily understood from the following more detailed description taken in conjunction with the accompanying drawing, in which:
FIG. 1 is a schematic block diagram of a prior art REDFA;
FIG. 2 is a schematic of an optical fiber 12' showing an input segment 12i, an adiabatic coupling segment 12a, and a low M² terminal segment 12t, in accordance with one embodiment of our invention;
FIG. 3 is a schematic of an optical fiber 12" showing, in addition, another low M² segment 12m disposed between a pair of adiabatic coupling segments 12a₂ and 12a₃, in accordance with another embodiment of our invention;
FIG. 4A is a schematic cross-sectional view of an REDF taken through its axis of propagation;
FIG. 4B is a schematic transverse refractive index profile of a terminal segment of the REDF shown in FIG. 4A, in accordance with yet another embodiment of our invention;
FIG. 4C is a schematic transverse refractive index profile of an input segment of the REDF shown in FIG. 4A, showing a pronounced dip in the profile at or near the center of the core region, in accordance with still another embodiment of our invention;
FIG. 4D is an expanded view of the pronounced dip in the schematic refractive index profile of the core region of FIG. 4C;
FIG. 5 is a graph of the core-cladding transverse refractive index step (Δn) versus radial position for an as-drawn fiber (Curve 5.1), a heat-treated fiber (Curve 5.2), and a uniform step index fiber (Curve 5.3);
FIG. 6 is a graph of normalized optical intensity of the fundamental transverse mode (LP₀₁) for an as-drawn fiber (Curve 6.1), a heat-treated fiber (Curve 6.2), and a uniform step index fiber (Curve 6.3); and
FIG. 7 is a graph of normalized optical intensity for a fiber with a pronounced center dip and for a uniform step index fiber versus radial position comparing the overlap between the fundamental transverse mode and the index profile of the as-drawn fiber (Curve 7.1) and the ideal uniform step index fiber (Curve 7.3) of the fiber designs of FIG. 5.

### DETAILED DESCRIPTION OF THE INVENTION

### General REDFA Structure

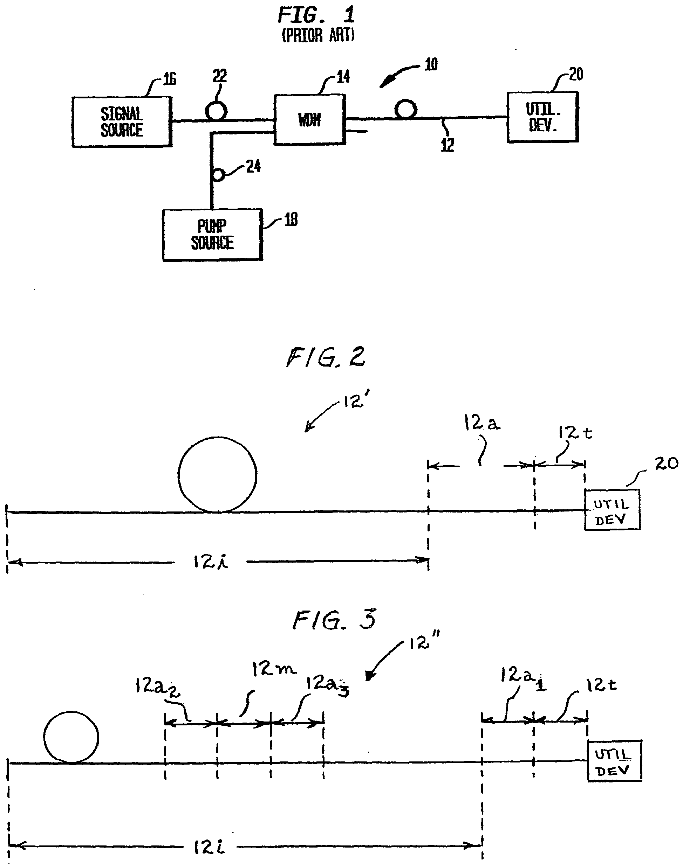

A typical REDFA 10, as shown in FIG. 1, comprises an REDF 12, which optically couples a coupling device 14 and a utilization device 20. In telecommunication applications device 14 is known as a wavelength division multiplexer; in high power non-telecommunications applications it is known as a pump-combiner. For simplicity, hereinafter we will describe our invention in the context of high power non-telecommunications applications. In this case, the pump-combiner 14 couples the outputs of an optical input signal source 16 and an optical pump source 18 into the REDF 12. The input signal source 16 generates a first-wavelength optical input signal, which is coupled to an input of a pump combiner 14 via a conventional fiber 22, whereas the pump source 18 generates a second-wavelength optical pump signal, which is coupled by a conventional fiber 24 to another input of pump combiner 14.

As is well known in the art, the pump signal generates a population inversion in the REDF 12, which amplifies the input signal from input source 16. The amplified input signal propagates along REDF 12 to utilization device 20. In high power applications the latter may include a myriad of well known devices or apparatuses; e.g., another REDFA, a beam collimator, a lens system, a work piece (e.g., for cutting or welding); whereas in telecommunications applications, utilization device 20 may include an optical receiver, an optical modulator, an optical coupler or splitter, or a piece of terminal equipment. Some of these may be coupled to the REDF 12 via a standard pigtail connector (not shown).

Illustratively, the input source 16 is a laser that generates a relatively low power optical input signal at wavelength in the amplification range of the rare earth species of REDF 12 , whereas the pump source 18 is a light emitting diode (LED) or an array of LEDs that generates a relatively high optical power (e.g., above about 150 mW) pump signal at a shorter center wavelength that produces the desired amplification of the input signal. Preferably, the REDF 12 is a ytterbium-doped fiber, the signal source 16 generates an input signal having a center wavelength of about 1080 nm, and the pump source 18 generates a pump signal at a center wavelength of about 915 nm, or alternatively at about 975 nm. We note here that a semiconductor laser may also be used as a pump source, but an LED, especially an array of LEDs, is preferred because more total light can be coupled into the fiber with an LED.

Although the REDFA of FIG. 1 depicts a common co-propagating pump configuration (i.e., the pump and input signals propagate in the same direction through the REDF), it is also possible to use a counter-propagating configuration (i.e., the pump and input signals propagate in opposite directions through the REDF). In addition, a multiplicity of REDFAs may be arranged in tandem, a scheme that is well known in the art for increasing the total gain of a high power multi-stage system. Pump energy may also be transversely coupled into the REDFA.

In addition, when provided with a suitable, well-known optical resonator (e.g., a pair of spaced apart fiber gratings) the REDF may function as a laser.

### Hybrid REDF Design

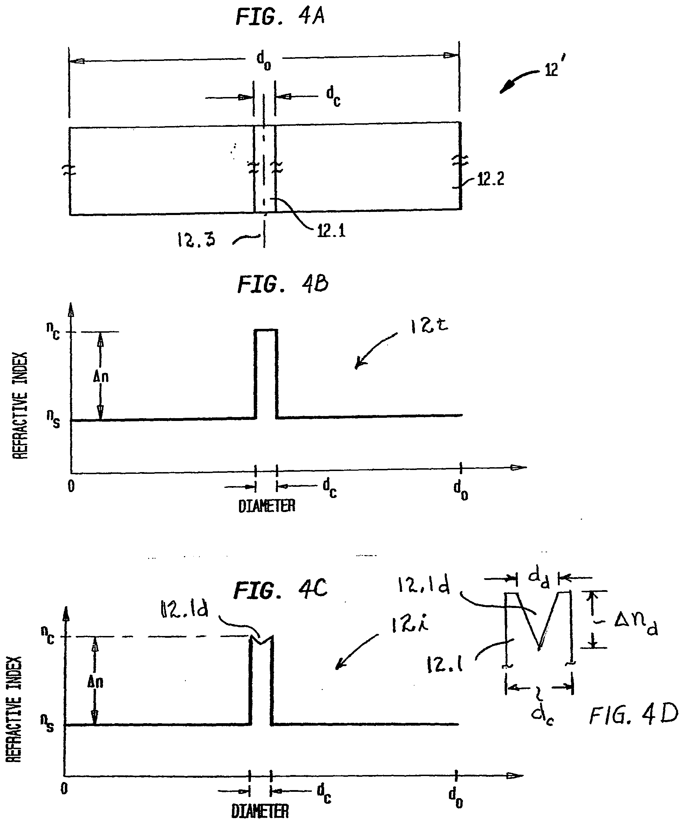

In accordance with one aspect of our invention, as shown in FIG. 4A, LMA REDF 12' includes a core region 12.1 of diameter d_{c} surrounded by a cladding region 12.2 of diameter do. We define the phrase *large mode area* (LMA) as follows: the core and cladding regions of a LMA fiber are configured to produce an effective mode area substantially larger then that of a conventional single mode fiber. For example, at a wavelength of about 1080 nm a conventional single mode fiber illustratively has a mode area of about 50 µm², but at the same wavelength a LMA fiber might have a mode area of about 100 µm². Similarly, at a wavelength of about 1550 nm a conventional single mode fiber illustratively has a mode area of about 80 µm², but at the same wavelength a LMA fiber might have a mode area of about 160 µm². Although these illustrations indicate that a LMA fiber has mode area twice as large as a single mode fiber at the same wavelength, other ratios may also be suitable depending the particular application of the LMA REDF and the performance desired.

The refractive index of the core region 12.1 is higher than that of the cladding region 12.2, with the difference in index being designated Δn. Although not shown, it is well known that the cladding may include an inner (*depressed*) cladding region and an outer cladding region, with the refractive index of the outer cladding region being between that of the core and the inner cladding region.

In either case, the core and cladding regions are configured to support the propagation of multiple transverse modes of input signal radiation propagating therein from source 16 (FIG. 1). In accordance with one aspect of our invention, the REDF 12' is a hybrid fiber, as shown in FIG. 2, in that it includes at least two axial segments that have different characteristics; namely, a LMA axial input segment 12i and a LMA axial terminal segment 12t adiabatically coupled to one another, for example, by means of a LMA axial adiabatic segment 12a. In addition, the hybrid fiber 12' may include a LMA terminal segment at its input end (not shown), at its output end (as shown in FIG. 2), or both.

More specifically, in one sense the terminal and input segments have different characteristics in that they have different transverse refractive index profiles, as shown in FIGs. 4B and 4C, respectively. In general the transverse refractive index profile of the core region of the terminal segment 12t is more uniform than that of the input segment 12i. The degree of uniformity is measured by the high frequency content of, for example, the Fourier transform of the profile shape. Thus, a profile whose Fourier transform contains fewer high frequency components is considered to be more uniform than a profile whose Fourier transform contains more high frequency components. Visual observation of the relative uniformity of simple profiles is often consistent with this type of quantitative analysis; for example, FIG. 4B shows that the profile of the core region of the terminal segment 12t exhibits an essentially constant transverse refractive index, and therefore has fewer high frequency components in its Fourier transform, whereas FIG. 4C shows that the profile of the core region of the input segment 12i exhibits a pronounced central dip in transverse refractive index, and therefore has more high frequency components in its Fourier transform. Thus, as shown in FIG. 4C, in the input segment 12i the transverse refractive index profile of the core region 12.1 is not radially uniform; that is, the index profile exhibits a pronounced dip 12.1d where the transverse index at or near the center of the core region 12.1 decreases by an amount Δn_{d}, as shown in FIG. 4D In contrast, in the terminal segment 12t the index profile of the core region is more nearly uniform (or radially constant), as shown in FIG. 4B.

In addition, the input segment 12i is configured to support more than one transverse mode.

In designing the features of the pronounced transverse refractive index dip 12.1d we prefer that the magnitude of Δn_{d} of the dip should be no greater than about 100% of Δn, the core-to-cladding index difference. The size of Δn depends on the rare earth dopant of the REDF as well as any index-altering dopants such as Ge, P, Al or F that might be added to the core and/or cladding regions; e.g., in Yb-doped fibers Δn ∼ 0.005, whereas in Er:Yb doped fibers Δn ∼ 0.01 At the opposite extreme, the magnitude of the dip should not be smaller than about 5% of Δn. The lower end of the range is dictated primarily by the need to perturb sufficiently the transverse mode shape from pure Gaussian, as discussed below. On the other hand, the width or diameter d_{d} of the dip should be larger than approximately the smallest wavelength of light used in the system (e.g., larger than the pump wavelength, which is typically shorter than the signal wavelength). At the opposite extreme, the maximum width d_{d} of the dip may be equal to the diameter d_{c} of the core region 12.1, but typically is about d_{c}/3. The object of these conditions is that the light "see" the perturbation in refractive index produced by the dip. In addition, although the dip is depicted as being conical, other geometric shapes (e.g., cylindrical) as well as more complex shapes, may also be suitable.

In another sense, the terminal and input segments have different characteristics in that their M² parameters are different from one another, where M² defines the similarity that the fundamental transverse mode of the fiber has to an ideal Gaussian function, as described by P. A. Belanger, Optical Engineering, Vol. 32. No. 9, pp. 2107-2109 (1993), which is incorporated herein by reference. (Although this paper defines M² for LP₀₁ fundamental mode of a step-index optical fiber, the definition is valid for all optical fibers, including those with a center dip in the transverse refractive index profile of the type described herein.) In particular, the input segment 12i is characterized by a parameter M₁², the terminal segment 12t is characterized by a parameter M₂², and the following inequalities are satisfied: M₁² > 1.0 and M₂² << M₁². In a preferred embodiment, M₁² >> 1.0 and M₂² ∼ 1.0. In theory M² may be arbitrarily large, but in practice M² for REDFs is typically in the range, 1 < M² < 10, approximately. Moreover, M² ∼ 1.06 is typically considered to be small in the sense of M₂² ∼ 1.0, for example, whereas M² ∼ 1.3 is considered to be large in the sense of M₁² >> 1.0, for example.

In addition the input segment 12i and the terminal segment 12t are coupled to one another adiabatically; for example, by means of a LMA adiabatic segment 12a, as shown in FIG. 2. In general such couplers insure that energy propagating in a particular transverse mode in the input segment is not significantly coupled into other transverse modes in the terminal segment, and conversely. Adiabatic coupling techniques and designs, which are well known in the art, include physically tapering the core regions so that the diameter smoothly increases (or decreases) in an axial direction along the coupling region, or chemically graduating the concentration of dopants so that their density increases (or decreases) gradually in an axial direction along the coupling region. In the latter case, a preferred technique involves (i) heating the REDF (e.g., with a conventional torch) to cause dopants in the fiber to diffuse, and (ii) controllably changing the amount of heat applied to the fiber in accordance with the longitudinal position of the torch along the fiber, so that the desired distribution of dopants is achieved.

The combination of the design of the M² parameter of the segments and the use of an adiabatic transition between them improves the coupling of the fundamental transverse mode, and significantly decreases coupling to higher order transverse modes, from the input segment to the terminal segment.

Another advantage of our invention is that the foregoing principles can be applied even in the absence of a fusion splice (a typical prior art approach to coupling different single mode fibers), for example, when coupling between the terminal segment of an REDF and a bulk (non-fiber) optical element (e.g., a telescope) is achieved in free space with the use of a suitable lens or lens system.

In a typical silica-based REDF well known in the art for operation in the wavelength ranges discussed above, the core region 12.1 is doped with at least one rare earth element (e.g., Er, Yb, Tb, Tm, Nd, and/or Pr) and one or more refractive-index-altering substances such as Ge, P or Al (to increase the index) or F (to decrease the index). The cladding region 12.2 may be pure silica, or it may also be doped. Illustratively the doping levels are chosen so that the index step Δn between the core and cladding ranges from about 0.005 to 0.01 depending on the dopants used, as discussed previously, and the index dip Δn_{d} in the input segment is about the same size as Δn.

Moreover, for the fiber to support multiple transverse modes the core diameter d_{c} is illustratively about 20 µm. The outer diameter do of such fibers is typically in the approximate range of 125 µm to 600 µm. In addition, it is apparent that the input segment 12i is a major fraction of the total length of fiber 12', whereas the terminal segment 12t is a relatively smaller fraction; e.g., the terminal segment is illustratively less than about 500 µm long, whereas the input segment is illustratively on the order of 1 m or 1 km long.

### Fiber Termination Treatment

As mentioned above, an elevated M² optical fiber can be locally heated to induce dopant diffusion that locally decreases the fiber's M². Heating a fiber to sufficient temperatures (for example, near or above fusion splicing temperatures of about 2000°C) induces substantial diffusion of the index-altering dopants, thereby inducing significant changes in the fiber's transverse refractive index profile. Such dopant diffusion is employed to suppress center dips, ridges, or other refractive index profile features that increase the M² of the fundamental LP₀₁ mode. In many (but not all) implementations of our invention, the MFD (mode field diameter) of the fundamental transverse LP₀₁ mode actually *decreases* following heat-induced diffusion.

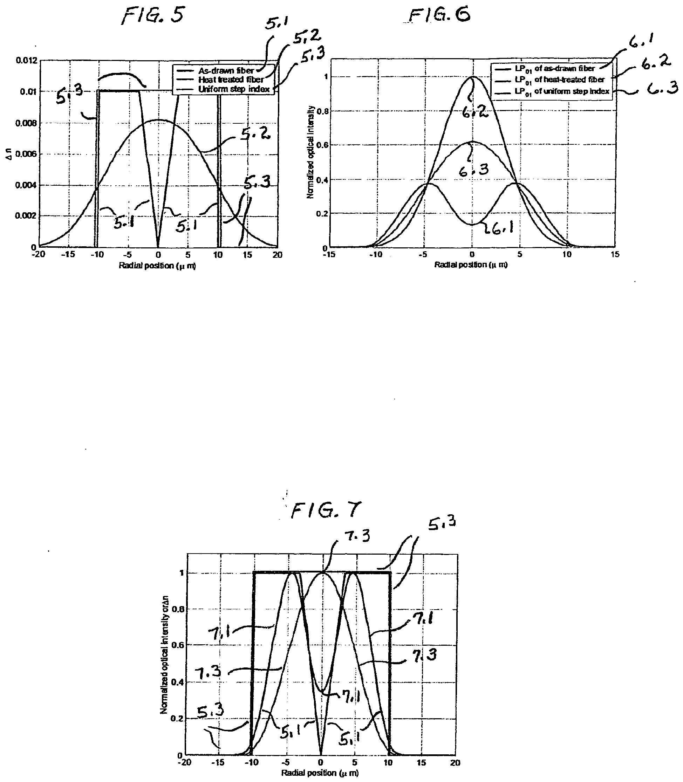

In our invention, the drawn optical fiber is locally heated to a high temperature (>> 1200°C) to induce dopant diffusion that suppresses the center-dip or other features in the refractive index profile that elevate the M² of the fiber's LP₀₁ fundamental mode. Curve 5.1 of FIG. 5 depicts a theoretical as-drawn fiber index profile for an Er:Yb doped fiber whose initial MFD (using the conventional "Petermann II" definition) is 13.4 µm and whose initial M²∼1.32. A profound center-dip is visible in this simulated refractive index profile. Heating such a fiber design to about 2100°C for about 25 seconds is expected to modify the refractive index profile (Curve 5.2) such that the final MFD is 13.3 µm and the initial M² is reduced (improved) to about 1.0. Applying this modification to the terminal segment 12t of fiber 12' (FIG. 2) is expected to significantly improve the coupling efficiency into or out of this fiber, regardless of the coupling technology used (conventional fusion splicing, connectorization, free-space coupling, GRIN fiber lenses, etc.). The corresponding transverse LP₀₁ mode field shapes (intensity fields) are depicted in FIG. 6.

For this particular example, it is important to note that for a given amount of power guided in the LP₀₁ fundamental mode, the peak optical intensity of the as-drawn fiber is only about 37% of the peak optical intensity occurring in the heat-treated fiber. Therefore, if an optical fiber designed with the transverse refractive index profile depicted in FIG. 5 (Curve 5.1) is terminated at each end with a segment 12t locally heat treated to induce the diffused index profile depicted in FIG. 5 (Curve 5.2), the peak optical intensity experienced in the majority of the fiber is expected to be relatively low so that the threshold for the onset of undesirable nonlinear optical effects (e.g., stimulated Brillouin scattering, or stimulated Raman scattering) will be relatively high. Meanwhile, the coupling efficiency will be excellent at the fiber termination points because M² in the terminal segment 12t approaches 1.0. The optical intensity is expected to be elevated only in the short (< about 500 µm) regions of heat-treated terminal segments of fiber. Since deleterious non-linear optical effects scale with the peak intensity as well as the length of the fiber segment, elevated optical intensity can be tolerated over short terminal segments of fiber.

Fiber terminations can be heat treated using a conventional fusion splicer. If the fiber is cleaved or polished inside the heat-treated region, then free-space coupling (for example, with conventional bulk lenses) can be used to obtain efficient optical coupling to the LP₀₁ fundamental transverse mode of a fiber whose as-drawn LP₀₁ mode field shape is very non-Gaussian. Alternatively, heat treatment can be incorporated as part of a fusion splicing process. The predicted coupling loss between a Gaussian field matching the MFD of the as-drawn (unheated) fiber in FIG. 6 (Curve 6.1) is about 0.7 dB, whereas the corresponding predicted coupling loss is less than about 0.01 dB for the heat treated fiber (Curve 6.2). Moreover, over 10% of the Gaussian energy will be coupled into the undesirable LP₀₂ mode of the as-drawn fiber, whereas the amount of energy coupled into the LP₀₂ mode of the heat-treated fiber is unmeasureable.

In order to ensure that energy is not lost from the LP₀₁ fundamental mode in the transition region between the heat-treated and as-drawn regions of the fiber, the transition should be made gradual and adiabatic, as discussed previously. The change in the refractive index profile in the transition region must be very gradual along its length. When producing the transition region by heat-induced dopant diffusion, a gradual transition can be achieved by varying the amount of heat applied to the transition region along its length, for example by choosing a broad (i.e., fanned out) heat source or scanning a more focused heat source along the transition region. How gradually this change must occur depends upon the details of the index profiles and the operating wavelength, according to principles well known in the art. Numerical simulations based on refractive index profiles as well as empirical process optimization can be readily employed to find suitable heating conditions for which the transition losses are minimized.

### Theory of Operation

When the fundamental transverse mode of a LMA fiber has an M² > 1.0. its coupling losses (free-space or fusion splice) are elevated, and the fundamental transverse mode input signal emerging from the fiber cannot be readily tightly focused down to a small spot size or readily collimated. However, there are certain advantages to having an elevated M² (> 1.0). In particular, fibers whose fundamental transverse mode fields have larger values for M² exhibit larger effective mode areas and hence lower peak optical intensities than fibers with the same core diameters but lower M². Consequently, fibers with elevated M² exhibit higher thresholds for the onset of undesirable optical nonlinearities such as SBS (stimulated Brillouin scattering) and SRS (stimulated Raman scattering). In addition to this benefit, fibers with an elevated M² (for example, due to a pronounced center-dip in the core region refractive index, as shown in FIG. 4C for input fiber segment 12i) can exhibit superior overlap between the rare earth dopants in the core region and the fundamental transverse mode field of the input signal propagating in the core region. Therefore, the amplification efficiency of the fundamental transverse mode can be increased and the amplification of undesirable higher order transverse modes can be decreased by designing a fiber with an elevated M².

These advantages are evident in FIGs. 5-6, which compare three LMA fibers: a theoretical as-drawn fiber having an elevated M² (curves 5.1, 6.1); a theoretical uniform step index fiber also having an elevated M² (Curves 5.3, 6.3); and a fiber heated-treated to reduce its M² (Curves 5.2, 6.2). Their refractive index profiles are compared in FIG. 5, and their corresponding fundamental LP₀₁ transverse mode optical intensity profiles at 1550 nm are compared in FIG. 6. The optical intensity in the as-drawn and step index fibers has been normalized to the peak intensity in the heat-treated portion of the fiber so that they both represent the same amount of optical power.

More specifically, the as-drawn fiber, which corresponds, for example, to the input fiber segment 12i of FIG. 2, exhibits a pronounced central dip (as quantified previously) in the core region transverse refractive index, and consequently an elevated fundamental mode M² of about 1.32 and a relatively large effective modal area of about 259 µm². Both the as-drawn fiber and the uniform step index fiber had Δn ∼ 0.01 and d_{c} ∼ 20 µm. However, the uniform step index fiber had a fundamental mode M² of about 1.05 and a reduced effective area of about 200 µm². FIG. 6 compares the normalized intensity distributions to the index profiles for these fibers in order to illustrate the superior overlap between the core region index profile and the intensity profile of the fiber with elevated M².

On the other hand, in the heat-treated fiber, which corresponds, for example, to the terminal fiber segment 12t of FIG. 2, the heat treatment has an improved (reduced) M² from 1.32 to about 1.0, reduced the effective modal area from 259 µm² to 139 µm², increased the peak optical intensity from about 0.37 to 1.0, and did not substantially alter the well-known "Petermann II" MFD (about 13.3 µm for both the as-drawn and heat-treated fibers). The index profile and corresponding normalized intensity distribution for an ideal uniform step index profile is also shown (Curves 5.3, 6.3) for comparison.

Finally, FIG. 7 compares of the overlap between the fundamental transverse mode and the refractive index profile of the as-drawn fiber (Curves 5.1, 7.1) and an ideal uniform step index fiber (Curves 5.3, 7.3) described above in conjunction with FIGs. 5-6. The index profiles and the optical intensities are individually normalized to themselves. FIG. 7 shows that a substantial portion of the outer core region of the step index fiber experiences a relatively low optical intensity, whereas a larger fraction of the as-drawn fiber experiences a higher optical intensity. Therefore, the as-drawn fiber has better overlap between the transverse mode field and the rare earth dopants, which means that the as-drawn fiber also exhibits better amplification efficiency.

### Alternative Embodiments

It is to be understood that the above-described arrangements are merely illustrative of the many possible specific embodiments that can be devised to represent application of the principles of the invention. Numerous and varied other arrangements can be devised in accordance with these principles by those skilled in the art without departing from the spirit and scope of the invention.

In particular, as shown in FIG. 3, a LMA optical fiber 12" may include a LMA intermediate segment 12m located at a position between the ends of the fiber in addition to the LMA terminal segment 12t, which is adiabatically coupled to input segment 12i by means of LMA adiabatic coupler 12a₁. The intermediate segment 12m is also adiabatically coupled to input segment 12i, illustratively by means of LMA adiabatic couplers 12a₂ and 12a₃. Like the terminal segment 12i, the intermediate segment 12m has a fundamental transverse mode M² that is less than that of the input segment 12i and is preferably close to 1.0. One application of such an intermediate segment 12m is to filter out undesirable high order transverse modes.

In addition, although we have described our invention in the context of EDFA applications, those skilled in the art will readily recognize its application can be extended to any apparatus that requires coupling to an EDF (e.g., a fiber laser).

## Claims

1. A multi-transverse-mode rare-earth-doped optical fiber comprising:
a core region doped with at least one rare earth element, the cross-section of said core region having a transverse refractive index profile,
a cladding region adjacent said core region,
said core and cladding regions configured to support multiple transverse modes of optical signal radiation within said core region,
said fiber including first and second axial segments optically coupled to one another, wherein each of said segments supports more than one of said transverse modes, including the fundamental transverse mode,
within said first axial segment said profile not being radially uniform and being **characterized by** a radial dip in refractive index, and within said second segment said profile being more uniform than within said first segment,
wherein said first segment is **characterized by** a parameter M₁², and said second segment is **characterized by** a parameter M₂²,where M² defines the similarity that the fundamental transverse mode of said fiber has to an ideal Gaussian function, and wherein M₁² > 1.0 and M₂² << M₁², and
wherein said segments are adiabatically coupled to one another.

2. The fiber of claim 1, wherein M₁² >> 1.0 and M₂² ∼ 1.0.

3. The fiber of claim 1, wherein said first segment comprises a major portion of the length of said fiber, and said second segment comprises a terminal portion of said fiber.

4. The fiber of claim 3, wherein said fiber includes a third axial segment optically coupled to said first segment, said profile of said third segment being more uniform than that of said first segment and being adiabatically coupled to said first segment, said second segment being located at one end of said first segment and said third segment being located at the opposite end of said first segment.

5. The fiber of claim 1, wherein said profile of said core region exhibits a dip in refractive index of Δn_{d}, which is approximately 5-100% of the difference Δn in transverse refractive index between said core region and said cladding region.

6. The fiber of claim 1, wherein fiber is configured to propagate said signal radiation in the fundamental transverse mode.

7. The fiber of claim 1, wherein said first segment comprises a major portion of the length of said fiber, and said second segment comprises an intermediate portion of said fiber.

8. The fiber of claim 1, wherein said core and cladding regions are configured to form a large mode area fiber.

9. An optical amplifier comprising:
an optical fiber according to claim 1 for amplifying said signal radiation in response to optical pump energy applied thereto,
a source of said pump energy, and
a coupler for coupling said pump energy and said signal radiation into said optical fiber.

10. The amplifier of claim 9, wherein said signal radiation has a first center wavelength and said source of pump energy comprises a LED for generating an optical pump signal having a second center wavelength.

11. A high power optical amplifier comprising:
a multi-transverse-mode, large-mode-area hybrid optical fiber according to claim 1,
wherein said core region is configured to amplify signal radiation propagating therein in response to optical pump energy applied thereto,
wherein said segments are adiabatically coupled to one another so that energy propagating in particular transverse mode in said first segment is not significantly coupled into other transverse modes in said second segment, and
wherein said second segment is located at either an input end of said first segment, at an output end of said first segment, or both,
a LED for generating said optical pump energy at a center wavelength different from that of said signal radiation, and
a pump combiner for coupling said pump energy into said fiber.

12. The amplifier of claim 11, wherein M₁² >> 1.0 and M₂² ∼ 1.0.

## Patentansprüche

1. Eine mit seltenen Erden dotierte optische Mehrfach-Transversalmode-Faser, die folgende Merkmale aufweist:
eine mit zumindest einem Seltenerdelement dotierte Kernregion, wobei der Querschnitt der Kernregion ein Transversalbrechungsindexprofil aufweist,
eine neben der Kernregion befindliche Mantelregion,
wobei die Kern- und die Mantelregion dazu konfiguriert sind, mehrere Transversalmoden einer optischen Signalstrahlung in der Kernregion zu unterstützen,
wobei die Faser ein erstes und eine zweites axiales Segment umfasst, die optisch miteinander gekoppelt sind, wobei jedes der Segmente mehr als eine der Transversalmoden, einschließlich der Grundtransversalmode, unterstützt,
wobei in dem ersten axialen Segment das Profil nicht radial einheitlich ist und durch einen radialen Einbruch des Brechungsindex gekennzeichnet ist, und in dem zweiten Segment das Profil einheitlicher ist als in dem ersten Segment,
wobei das erste Segment durch einen Parameter M₁² gekennzeichnet ist und das zweite Segment durch einen Parameter M₂² gekennzeichnet ist, wobei M² die Ähnlichkeit, die die Grundtransversalmode der Faser mit einer idealen Gauß-Funktion hat, definiert, und wobei M₁² > 1,0 und M₂² « M₁² und
wobei die Segmente adiabatisch miteinander gekoppelt sind.

2. Die Faser gemäß Anspruch 1, bei der M₁² » 1,0 und M₂² ∼ 1,0.

3. Die Faser gemäß Anspruch 1, bei der das erste Segment einen Hauptabschnitt der Länge der Faser aufweist und das zweite Segment einen Endabschnitt der Faser aufweist.

4. Die Faser gemäß Anspruch 3, wobei die Faser ein mit dem ersten Segment optisch gekoppeltes drittes axiales Segment umfasst, wobei das Profil des dritten Segments einheitlicher ist als das des ersten Segments und adiabatisch mit dem ersten Segment gekoppelt ist, wobei sich das zweite Segment an einem Ende des ersten Segments befindet und sich das dritte Segment an dem gegenüberliegenden Ende des ersten Segments befindet.

5. Die Faser gemäß Anspruch 1, bei der das Profil der Kernregion einen Einbruch des Brechungsindex von Δn_{d} aufweist, der ungefähr 5-100% der Differenz Δn des Transversalbrechungsindex zwischen der Kernregion und der Mantelregion beträgt.

6. Die Faser gemäß Anspruch 1, wobei die Faser dazu konfiguriert ist, die Signalstrahlung in dem Grundtransversalmode auszubreiten.

7. Die Faser gemäß Anspruch 1, bei der das erste Segment einen Hauptabschnitt der Länge der Faser aufweist und das zweite Segment einen Zwischenabschnitt der Faser aufweist.

8. Die Faser gemäß Anspruch 1, bei der die Kern- und die Mantelregion dazu konfiguriert sind, eine Faser mit großer Modenfläche zu bilden.

9. Ein optischer Verstärker, der folgende Merkmale aufweist:
eine optische Faser gemäß Anspruch 1 zum Verstärken der Signalstrahlung ansprechend auf eine daran angelegte optische Pumpenergie,
eine Quelle der Pumpenergie und
einen Koppler zum Koppeln der Pumpenergie und der Signalstrahlung in die optische Faser.

10. Der Verstärker gemäß Anspruch 9, bei dem die Signalstrahlung eine erste Mittenwellenlänge aufweist und die Quelle der Pumpenergie eine LED zum Erzeugen eines optischen Pumpsignals mit einer zweiten Mittenwellenlänge aufweist.

11. Ein optischer Hochleistungsverstärker, der folgende Merkmale aufweist:
eine optische Mehrfach-Transversalmode-Hybridfaser Faser mit großer Modenfläche gemäß Anspruch 1,
bei der die Kernregion dazu konfiguriert ist, eine Signalstrahlung, die sich in derselben ausbreitet, ansprechend auf eine an dieselbe angelegte optische Pumpenergie zu verstärken,
bei der die Segmente adiabatisch miteinander gekoppelt sind, so dass Energie, die sich insbesondere in der Transversalmode in dem ersten Segment ausbreitet, nicht auf bedeutende Weise in andere Transversalmoden in dem zweiten Segment gekoppelt ist, und
bei der sich das zweite Segment entweder an einem Eingangsende des ersten Segments oder an einem Ausgangsende des ersten Segments oder an beiden befindet,
eine LED zum Erzeugen der optischen Pumpenergie bei einer Mittenwellenlänge, die sich von der der Signalstrahlung unterscheidet, und
einen Pumpenkombinierer zum Koppeln der Pumpenergie in die Faser.

12. Der Verstärker gemäß Anspruch 11, bei dem M₁² >> 1,0 und M₂² ∼ 1,0.

## Revendications

1. Fibre optique multimode transversal dopée aux terres rares, comprenant:
une région de noyau dopée avec au moins un élément de terres rares, la section de ladite région de noyau présentant un profil d'indice de réfraction transversal,
une région de gaine adjacente à ladite région de noyau,
lesdites régions de noyau et de gaine étant configurées pour supporter de multiples modes transversaux de rayonnement de signal optique dans ladite région de noyau,
ladite fibre comprenant un premier et un deuxième segment axial couplés optiquement l'un à l'autre, où chacun desdits segments supporte plus d'un desdits modes transversaux, y compris le mode transversal fondamental,
dans ledit premier segment axial, ledit profil n'étant pas uniforme radialement et étant **caractérisé par** un creux radial dans l'indice de réfraction, et dans ledit deuxième segment, ledit profil étant plus uniforme que dans ledit premier segment,
dans laquelle ledit premier segment est **caractérisé par** un paramètre *M*₁², et ledit deuxième segment est **caractérisé par** un paramètre *M*₂², où *M*² définit la similitude que présente le mode transversal fondamental de ladite fibre avec une fonction gaussienne idéale, et où *M*₁² > 1.0 et *M*₂² << *M*₁², et
dans laquelle lesdits segments sont couplés de manière adiabatique l'un à l'autre.

2. Fibre selon la revendication 1, dans laquelle *M*₁² >> 1.0 et *M*₂²∼1.0.

3. Fibre selon la revendication 1, dans laquelle ledit premier segment comprend une partie principale de la longueur de ladite fibre et ledit deuxième segment comprend une partie terminale de ladite fibre.

4. Fibre selon la revendication 3, dans laquelle ladite fibre comporte un troisième segment axial couplé optiquement audit premier segment, ledit profil dudit troisième segment étant plus uniforme que celui dudit premier segment et étant couplé de manière adiabatique audit premier segment, ledit deuxième segment étant situé à une extrémité dudit premier segment et ledit troisième segment étant situé à l'extrémité opposée dudit premier segment.

5. Fibre selon la revendication 1, dans laquelle ledit profil de ladite région de noyau présente un creux dans l'indice de réfraction Δ*n_{d}* qui est d'environ 5 à 100% de la différence Δ*n* de l'indice de réfraction transversal entre ladite région de noyau et ladite région de gaine.

6. Fibre selon la revendication 1, la fibre étant configurée pour propager ledit rayonnement de signal dans le mode transversal fondamental.

7. Fibre selon la revendication 1, dans laquelle ledit premier segment comprend une partie principale de la longueur de ladite fibre, et ledit deuxième segment comprend une partie intermédiaire de ladite fibre.

8. Fibre selon la revendication 1, dans laquelle lesdites régions de noyau et de gaine sont configurées pour former une fibre à grande surface de mode.

9. Amplificateur optique, comprenant:
une fibre optique selon la revendication 1, pour amplifier ledit rayonnement de signal en réponse à l'énergie de pompage optique y appliquée,
une source de ladite énergie de pompage, et
un coupleur destiné à coupler ladite énergie de pompage et ledit rayonnement de signal dans ladite fibre optique.

10. Amplificateur selon la revendication 9, dans lequel ledit rayonnement de signal présente une première longueur d'onde centrale et ladite source d'énergie de pompage comprend une diode électroluminescente pour générer un signal de pompage optique présentant une deuxième longueur d'onde centrale.

11. Amplificateur optique de haute puissance, comprenant:
une fibre optique hybride multimode transversal à grande surface de mode selon la revendication 1,
dans laquelle ladite région de noyau est configurée pour amplifier un rayonnement de signal s'y propageant en réponse à l'énergie de pompage optique y appliquée,
dans laquelle lesdits segments sont couplés de manière adiabatique l'un à l'autre, de sorte que la propagation d'énergie en particulier en mode transversal dans ledit premier segment ne soit pas couplée de manière significative dans d'autres modes transversaux dans ledit deuxième segment, et
dans lequel ledit deuxième segment est situé soit à une extrémité d'entrée dudit premier segment, soit à une extrémité de sortie dudit premier segment, soit aux deux,
une diode électroluminescente pour générer ladite énergie de pompage optique à une longueur d'onde centrale différente de celle dudit rayonnement de signal, et
un combineur de pompe destiné à coupler ladite énergie de pompage dans ladite fibre.

12. Amplificateur selon la revendication 11, dans lequel *M*₁² >> 1.0 et *M*₂²∼1.0.
